# EUROPEAN PATENT SPECIFICATION

(11) **EP 3 021 663 B1**
(45) Date of publication and mention of the grant of the patent: **18.01.2023**
(21) Application number: 14830365.4
(22) Date of filing: 11.10.2014
(51) Int. Cl.: A01K 67/027, C07K 14/775

(54) **TRANSGENIC MOUSE EXPRESSING HUMAN LIPOPROTEIN (A) WITH DISABLED VITAMIN C GENE**
MENSCHLICHES LIPOPROTEIN EXPRIMIERENDE TRANSGENE MAUS MIT DEAKTIVIERUNG DES VITAMIN-C-GENS
SOURIS TRANSGÉNIQUE EXPRIMANT LA LIPOPROTÉINE (A) HUMAINE AVEC GÈNE DE LA VITAMINE C DÉSACTIVÉ

(30) Priority: 05.10.2014 US 201414506674
(43) Date of publication of application: 25.05.2016
(73) Proprietor: Rath, Matthias W., Aptos CA 95003 (US)
(72) Inventor: RATH, Matthias, W., Henderson, NV 89044 (US); NIEDZWIECKI, Aleksandra, Henderson, NV 89044 (US); CHA, John, Chang-Eun, Sanmateo, CA 94403 (US)
(74) Representative: Féaux de Lacroix, Stefan
(86) International application number: PCT/US2014/060195
(87) International publication number: WO 2016/057050

(56) References cited:
- WO-A1-99/35241
- US-A1- 2015 074 837
- Y. NAKATA ET AL: "Vulnerable Atherosclerotic Plaque Morphology in Apolipoprotein E-Deficient Mice Unable to Make Ascorbic Acid", CIRCULATION, vol. 105, no. 12, 26 March 2002 (2002-03-26), pages 1485-1490, XP055295478, US ISSN: 0009-7322, DOI: 10.1161/01.CIR.0000012142.69612.25
- EUAN J. RODGER ET AL: "Proteomic Analysis of Aortae from Human Lipoprotein(a) Transgenic Mice Shows an Early Metabolic Response Independent of Atherosclerosis", PLOS ONE, vol. 7, no. 1, 19 January 2012 (2012-01-19), page e30383, XP055273503, DOI: 10.1371/journal.pone.0030383
- CHA J, NIEDZWIECKI A, RATH M: "Hypoascorbemia induces atherosclerosis and vascular deposition of lipoprotein(a) in transgenic mice.", AM J CARDIOVASC DIS. 2015 MAR 31, vol. 5, no. 1, 20 March 2015 (2015-03-20), pages 53-62, XP055295448,
- JOHN CHA, M. WAHEED ROOMI, ALEKSANDRA NIEDZWIECKI, MATTHIAS RATH: "Abstract 2288: Lipoprotein(a) and vitamin C affect the development of breast cancer tumors in Lp(a)+; Gulo-/- mice", CANCER RESEARCH; PROCEEDINGS: AACR 106TH ANNUAL MEETING 2015; APRIL 18-22, 2015; PHILADELPHIA, PA, vol. 75, no. 15 Suppl, 1 August 2015 (2015-08-01), XP002760785,
- JOHN CHA ET AL: "Lipoprotein(a) and vitamin C affect the development of breast cancer tumors in Lp(a)+; Gulo-/- mice", INTERNATIONAL JOURNAL OF ONCOLOGY, SEPTEMBER 2016, vol. 49, no. 3, 1 July 2016 (2016-07-01), XP002760783,
- RODGER ET AL.: 'Proteomic analysis of aortae from human lipoprotein(a) transgenic mice shows an early metabolic response independent of atherosclerosis.' PLOS ONE. vol. 7, no. 1, 2012, page E30383, XP055273503
- MAEDA ET AL.: 'Aortic wall damage in mice unable to synthesize ascorbic acid.' PROC NATL ACAD SCI USA vol. 97, no. 2, 2000, pages 841 - 6, XP055273505
- CSONT ET AL.: 'Hypercholesterolemia increases myocardial oxidative and nitrosative stress thereby leading to cardiac dysfunction in apoB-100 transgenic mice.' CARDIOVASC RES. vol. 76, no. 1, 2007, pages 100 - 9, XP022264260
- DATABASE GENBANK [Online] 11 November 2003 'Mus musculus BAC clone RP24-136A8 from chromosome 14', XP055273507 Retrieved from NCBI Database accession no. AC126444.3
- N. MAEDA ET AL: "Aortic wall damage in mice unable to synthesize ascorbic acid", PROCEEDINGS OF THE NATIONAL ACADEMY OF SCIENCES, vol. 97, no. 2, 18 January 2000 (2000-01-18), pages 841-846, XP055273505, US ISSN: 0027-8424, DOI: 10.1073/pnas.97.2.841
- John Cha ET AL: "Hypoascorbemia induces atherosclerosis and vascular deposition of lipoprotein(a) in transgenic mice", , 31 March 2015 (2015-03-31), pages 53-62, XP055295448, Retrieved from the Internet: URL:http://www.ncbi.nlm.nih.gov/pmc/articl es/PMC4447075/pdf/ajcd0005-0053.pdf [retrieved on 2016-08-15]

## Description

### FIELD OF TECHNOLOGY

This disclosure relates generally to a transgenic mouse that has been genetically altered to express human lipoprotein (a) and a disabled gene for the expression of Vitamin C. More specifically the two defining human proteins of lipoprotein (a), apolipoprotein (a) and apolipoprotein B-100 gene may be expressed either individually or in combination without the expression of Vitamin C gene. The dual transgenic mouse embryo referred to as Rath M Human Lipoprotein(a);Gulo(-/-) has the Jackson Stock# 912329, having been deposited in The Jackson Laboratory on April 8, 2013.

### BACKGROUND OF THE INVENTION

Cardiovascular disease is responsible for half of the deaths in the industrial world. Over the past decades a new risk factor for this disease has emerged, lipoprotein (a)(Lp (a)). Lp (a) is has been shown to be an independent risk factor for myocardial infarctions, (Rhoads GG et. al.,1986, Clarke et al, 2009) cerebrovascular disease ( Zenker G et. al.,1986) and other forms of cardiovascular disease. Furthermore, Lp(a) has been identified as a significant component of human atherosclerotic plaques (Rath M et. al., 1989). Aside from Niacin, there is currently no accepted effective treatment available in clinical cardiology to lower Lp(a) plasma levels or to prevent its deposition inside the vascular wall.

Lp(a) was discovered by Kare Berg in 1963 (Berg, K et. al., 1963). It is composed of a low-density-lipoprotein molecule (LDL) and apolipoprotein (a) (apo(a)), a glycoprotein attached to the structural protein of LDL, apolipoprotein B-100 (apo B), via disulfide bonds. The cDNA of apo(a) shows a strong homology with plasminogen containing multiple repeats of plasminogen kringle IV. Due to this homology apo(a) binds to fibrinogen/fibrin and attenuates fibrinolysis (McLean, J W et. al. 1987).

Lp(a) is primarily found in humans and subhuman primates and the appearance of the apo(a) gene was dated to about 40 million years ago, about the time of the divergence of the Old World and New World monkeys (McLean, et al. Nature, 1987). This was also the time point during evolution when the ancestor of man lost the ability for endogenous ascorbate synthesis due to a mutation in the gene encoding for gulonolactone oxidase (GULO), an essential enzyme for the conversion of glucose to ascorbate (vitamin C) (Chatterjee IB, 1973, Nikishimi M et al., 1991). GULO(-/-) mice are taught in Maeda et al., PNAS, 2000, 97(2):841-846 and in Nakata and Maeda, Circulation, 2002, 105:1485-1490.

A transgenic rabbit that expresses a functional lipoprotein (a) is disclosed in WO 99/35241. Lipoprotein (a) transgenic mice and proteomic analysis thereof is taught in Rodger et al., PLoS ONE, 2012, 7(1):e30383.

The significance of ascorbate deficiency in initiating the process of atherogenesis has recently been documented in mouse unable to express the gene for L-gulonolactone oxidase (GULO -/-) (Maeda N et. al., 2000).

Roy et. al. (2003, US 6512161) discusses several failed attempts to create animal models for expressing specifically Lp(a) in models such as rats, mouse and guinea pigs and state that they don't always represent human metabolism and human-related diseases. In their study they invented a rabbit model expressing human apo (a) and human apo B-100 genes. However, the transgenic rabbit developed by Roy et. al. (2003) also does not mimic the human physiology with respect to another key metabolic aspect: unlike humans, rabbits are able to produce their own Vitamin C.

There exists a need for a dual transgenic mammal model displaying these unique genetic features in order to develop new preventive and therapeutic approaches related to them.

### SUMMARY

The current invention is defined by the claims. Further disclosed, but not forming part of the present invention as such, is a method of making and using a dual transgenic mouse that possesses the genes for human apo (a) and/or apo B-100 and produces human (Lp(a) while, at the same time, being unable to produce vitamin C. It is disclosed that a third strain of transgenic mouse was obtained by crossbreeding the first knockout strain mouse and second strain of transgenic mouse expressing a set of genes that are human in nature, wherein the first strain is a knockout mouse possessing a non-functional L-gulonolactone oxidase (GULO) (GULO-/-) and hence produces no Vitamin C, wherein the second strain of mice expresses human apo (a) gene (apo (a) +) and produces apolipoprotein (a)(apo (a)), wherein the third strain of transgenic mice possesses non-functional L-gulonolactone oxidase (GULO-/-) gene and a functional human apo (a) gene (apo (a) +). Hence this third strain of mouse will not produce vitamin C but will produce human apo (a).

It is disclosed that a fifth strain of transgenic mouse was made by crossbreeding the first knockout strain mouse possessing non-functional L-gulonolactone oxidase (GULO-/-) gene and the fourth strain expressing human apo B-100 gene (apo (B-100) +), wherein the fifth strain of mouse possesses non-functional L-gulonolactone oxidase (GULO-/-) gene and a functional human apo B-100 gene (apo (B-100) +). Hence the fifth strain of transgenic mouse will not produce vitamin C and will produce human apolipoprotein B-100.

It is disclosed that third strain and fifth strain of transgenic mouse were crossbred to obtain a novel dual transgenic according to the invention that had a knockout GULO gene (GULO-/-), a functional human apo B-100 gene (apo( B-100) +) and a functional human apo (a) gene (apo (a) +). The novel dual transgenic mouse according to the invention will hence, produce apolipoprotein (a)(apo (a)) and/or apolipoprotein B-100 (apo B-100) as well as the complete lipoprotein(a) particle (Lp(a) and will not produce vitamin C. This novel double transgenic mouse model resembles the human system with respect to the inability of endogenous ascorbate synthesis and, congruently, the expression of apo(a), apo (B-100) as well as the complete lipoprotein(a) particle (Lp(a)). In the instant disclosure a mouse model is used, but other non-human mammals, that are not part of the invention, may be used and crossbreeding, insertion of genes or deletion of genes may be done to produce these dual transgenic non-human mammals to express or suppress human genes in any combination.

A mouse whose genome lacks the ability for endogenous ascorbate synthesis and - simultaneously - expresses a human apo (a) is disclosed.A dual transgenic mouse whose genome lacks the ability for endogenous ascorbate synthesis and - simultaneously - expresses a human apo B-100 is disclosed. A transgenic mouse whose genome lacks the ability for endogenous ascorbate synthesis and - simultaneously - produces a human Lp(a) is disclosed. A mouse model may be created by crossbreeding, gene insertion or other methods of molecular biology and/or genetic engineering.

In one embodiment, the novel dual transgenic mouse as defined in the claims may be used as a model for cardio vascular disease (CVD) study. In another embodiment, the dual transgenic mice as defined in the claims may be used as a model for use in a method for treating CVD like diseases. In another embodiment, the dual transgenic mouse as defined in the claims may be used as a model to test new and old drugs for use in a method to treat diseases associated with Lp(a) synthesis and lack of vitamin C production.

In one embodiment, the dual transgenic mouse model as defined in the claims may be used for testing effect of various drugs involved in ischemic heart disease, cardiovascular diseases, including coronary artery disease, cerebrovascular disease (stroke), renal vascular disease, peripheral vascular disease, aneurysms, thrombotic conditions, other forms of vascular disease, inflammatory conditions, as well as infectious diseases, neuroinflammatory and neurodegenerative diseases.

Disclosed herein, and not part of the invention, is a process for making a dual transgenic non-human mammal which lacks the ability for endogenous ascorbate synthesis and - simultaneously - is capable of producing human apolipoprotein(a) or human apolipoprotein B or Lp(a) comprising mating a first non-human mammal in which the ability for ascorbate synthesis has been genetically deleted with a second non-human mammal which has a genome encoding human apo (a) or human apo B-100 or both of these apolipoproteins in such a manner that they combine in vivo in said transgenic non-human mammal to produce the complete human lipoprotein(a) particle (Lp(a)).

A method for determining whether a compound can be used in a method for treating atherosclerosis or an undesirable plasma lipid profile (not according to the invention as such) is disclosed, comprising: a) comparing the lipid profile or state of atherosclerosis in a first transgenic non-human mammal fed a diet generally known to be atherogenic and treated with said compound, to the lipid profile or state of atherosclerosis in a second transgenic non-human mammal fed the same atherogenic diet but not treated with said compound; and determining the potential therapeutic effect of said compound based upon comparative evaluation of the lipid profile or state of atherosclerosis in said first and second transgenic non-human mammal; wherein said first and second transgenic non-human mammal each being a transgenic non-human mammal.

Further disclosed is the drug for use in a method for treating (not according to the present invention) wherein the drug to treat the effect of high Lp(a) in the absence or in presence of micronutrients such as vitamin C is observed using transgenic mouse that does not produce vitamin C and produces human Lp(a).

The composition, method, and treatment disclosed herein may be implemented in any means for achieving various aspects, and may be executed in a form suitable for the mammal. The present invention is defined in the claims.

### BRIEF DESCRIPTION OF DRAWINGS

Example embodiments are illustrated by way of example and not limitation in the Figures of the accompanying drawings, in which like references indicate similar elements and in which:
**Fig. 1** shows the scheme used for creating the dual transgenic mouse.
**Fig. 2** shows the expression of disulfide linked human apo(a) and human apoB-100 as assembled Lp(a) with a lipid globule in male and female transgenic mouse with both human apo(a) and human apo B-100 genes, but not in male and female transgenic mouse without both genes.
**Fig. 3** shows the expression of human apo(a) protein in female and male mice.
**Fig. 4** shows the expression of human apo B-100 protein in female and male transgenic mouse in LDL particles and Lp(a) particles.
**Fig. 5** shows the serum ascorbate level in transgenic female and male mouse (26-29 weeks old) supplemented with Vitamin C or deprived of Vitamin C in micromoles/liter (uM).
**Fig. 6** shows Serum Protein Immunofixation Electrophoresis (SPIFE) cholesterol profile of hypoascorbemic or fully ascorbate-supplemented Lp(a) + GULO (-/-) mouse.
Fig. 7 shows Immunofixation Electrophoresis (IFE) apo(a)-particle profile of hypoascorbemic or fully ascorbate-supplemented Lp(a) + GULO (-/-) mouse.
**Fig. 8** shows IFE Human Apo B-Particle profile of hypoascorbemic or fully ascorbate- supplemented Lp(a) + GULO (-/-) mouse.

Other features of the present embodiments as embraced by the claims will be apparent from the accompanying drawings and from the detailed description that follows.

### DETAILED DESCRIPTION

The invention is defined by the claims. It refers to a novel dual transgenic mouse. Generally disclosed is a method of crossbreeding a dual transgenic non-human mammal (not forming part of the present invention as such, may be a mouse or other animals) and the use of the dual transgenic mouse for assessing treatment method for cardiovascular and related diseases. The dual transgenic mouse expresses human apolipoprotein (a) and apolipoprotein B-100 genes and produces apolipoprotein (a) and apolipoprotein B-100 as well as complete human lipoprotein (a) particles in this mouse which, simultaneously, does not express L-gulonolactone oxidase (GULO -/-) and, consequently, does not produce vitamin C. This novel dual transgenic mouse is the ideal model for testing pharmaceutical compounds for treatment efficacy and usefulness for Lp(a) modulation with a variety of biological and/or pharmaceutical compounds, including but not limited to, nutrition, pharmaceutical drugs and treatment methods that affect human beings.

The invention is defined by the claims.

### CROSS BREEDING OF NON-HUMAN MAMMAL/MOUSE:

A non-human animal model may be created by crossbreeding, gene insertion or other methods of molecular biology and/or genetic engineering. In an exemplary example crossbreeding of a knock mouse and a specific human expressing gene containing mouse is disclosed to create a dual transgenic mouse.

**BALB/cBy-Gulo (-/-) mouse:** The strain, BALB/cBy-*Gulo^{sfx}*/J was a spontaneous mutation, mapped to the Gulonolactone oxidase locus, the gene for vitamin C synthesis. The GULO (-/-) strain mouse (the first knockout strain of transgenic mouse) was generated from heterozygous (hemizygous) GULO (+/-) breeders obtained from The Jackson Laboratory (Table 1). The mouse was bred under vitamin C supplementation until an adequate number of homozygous GULO (-/-) breeders were obtained.

**Table 1: GULO (-/-) strain description:**

| Allele Symbol | Gulo^{sfx} |
|---|---|
| Allele Name | spontaneous fracture |
| Allele Type | Spontaneous |
| Strain of Origin | BALB/cBy-Rasa3 |
| Gene Symbol and Name | Gulo, gulonolactone (L-) oxidase |
| Chromosome | 14 |
| Gene Common Name(s) | AU018375; BC028822; L-gulono-gamma-lactone oxidase; MGC:29968; MGC:37793; MGC:37880; cDNA sequence BC028822; expressed sequence AU018375; sfx; spontaneous fracture; |
| General Note | This spontaneous mutation appeared in a BALB/cBy-scat colony at The Jackson Laboratory. The scat and sfx mutations were separated from each other by backcrossing BALB/cBy-scat mouse to BALB/cBy mouse and observing F2 offspring for those that exhibited the sfx phenotype but not the scat phenotype. |
| Molecular Note | The mutation in the sfx mouse is a deletion that includes the entire Gulo gene. [MGI Ref ID J:95128] |

Maintenance of GULO (-/-) mouse: GULO (-/-) mouse are unable to synthesize their own vitamin C; therefore this nutrient needs to be present in the mouse diet. Vitamin C was provided in a double distilled drinking water containing 150mg/L ascorbic acid (Sigma) and 0.01 mM EDTA (Sigma) to stabilize vitamin C from degradation by interaction with trace metals. The water also contained 10g/L of sucrose in order to mask a taste offensive to mouse. Water was changed twice a week. In addition, these mouse received food fortified with 500ppm L ascorbyl-polyphosphate, the standard veterinary feed source of stable vitamin C milled at Test Diet as Modified Custom Lab Diet #5A38.

**Human Apo (a) mouse:** The Human apo(a) mouse (second strain of transgenic mouse) was obtained from the Mutant Mouse Regional Resource Centers (MMRRC), supported by the NIH. The strain, FVB/N-Tg(LPA, LPAL2, PLG)1Hgc/Mmmh, was created using a 270kb YAC that harbors human apo(a) and apo(a) like and plasminogen genes. The donor was Edward M. Rubin, M.D., Ph.D., Lawrence Berkeley National Laboratory. Founder mice were bred until sufficient number of apo(a)+ Gulo wildtype mice were obtained for crossbreeding. Genotyping for the transmission and presence of the transgene was performed at Transnetyx (Cordova, TN) upon tail clip tissue and transgenic mutants confirmed positive for apo(a) in the genome selected for cross-breeding.

**Human Apo B-100 mouse:** The human apoB-100 mouse (fourth strain of transgenic mammal/mouse) was obtained from Taconic Farms, Inc. under academic research agreement. The strain, B6.SJL-Tg(APOB)1102Sgy N20+?, or apoB-100 mouse, was developed by MacRae F. Linton et. al. of the Gladstone Institute of Cardiovascular Disease by microinjecting the human apolipoprotein B100 gene into C57BL/6J x SJL zygotes. The resultant mouse was backcrossed to C57BL/6 for 4 generations (N4). Taconic received stock from Xenogen Biosciences in May 1996. The mouse was maintained by backcrossing hemizygous Apo (B-100) mouse with C57BL/6NTac inbred mouse. Hemizygous mouse were bred to obtain homozygous Apo (B-100) mouse. Genotyping for transmission and presence of the transgene Human Apo (B-100) in the genome was performed at Transnetyx upon tail clip tissue and transgenic mutants selected for cross-breeding.

### Cross breeding steps leading to generating a mouse strain producing human Lp(a):

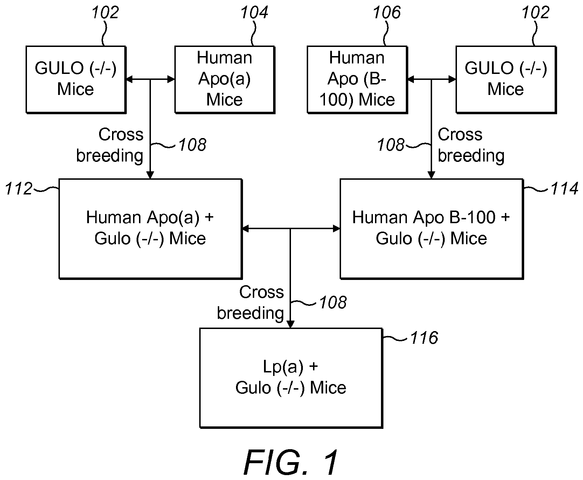

Fig. 1 shows the various steps used for crossbreeding several strains of mouse to obtain a dual transgenic mouse. The terms mouse and mice are used interchangeably and they all mean mouse in the instant specification. Crossbreeding (108) of human apo (a) mice (104) (the second strain of mammal) + GULO (-/-) (102) (a first knockout strain mammal) mice/mouse to produce a third strain of transgenic mammal/mouse that expresses the human apolipoprotein (a) gene (apo (a) +) and, simultaneously lacks the GULO gene (GULO-/-) (112) was performed. A GULO (-/-) (102) (a first knockout strain mammal) and human apo B-100 mouse represented as fourth strain of mammal (106) was crossbred (108) to produce a fifth strain of mouse that the fifth strain of mammal lacks the GULO gene (GULO-/-) and expresses the human apolipoprotein B-100 gene (apo (B-100) +) (114). Two transgenic founding strains were thus obtained for further crossbreeding:
- Human apo(a)+ GULO (-/-) mouse - third strain of transgenic mouse
- Human apo B-100+ GULO (-/-)mouse - fifth strain of transgenic mouse.

**Crossbreeding (108) the founding strains for obtaining mouse strain:human Lp(a)+ GULO (-/-) (116) :** Newly generated mouse breeders of human apo(a)+ GULO (-/-) mouse (112) and human apo B100+ GULO (-/-) (114) were subsequently crossed (108) with one another to generate the new mouse strain: human Lp(a) + GULO (-/-) mice (116) which had human apo(a)+ human ApoB-100+ GULO (-/-) , named as "Rath M Human Lipoprotein(a);Gulo (-/-)" strain (116). The dual transgenic mouse embryo referred to as Rath M Human Lipoprotein(a);Gulo(-/-) has the Jackson Stock# 912329, having been deposited in The Jackson Laboratory on April 8, 2013.

GENOTYPING: Genotyping for the GULO locus and its homozygosity was performed via Taqman FAM Probe Real Time-PCR at Transnetyx upon tail clip tissue derived DNA obtained using standard DNA isolation and PCR techniques. Transgene presence for human apo B-100 and human apo(a) were also conducted at Transnetyx.

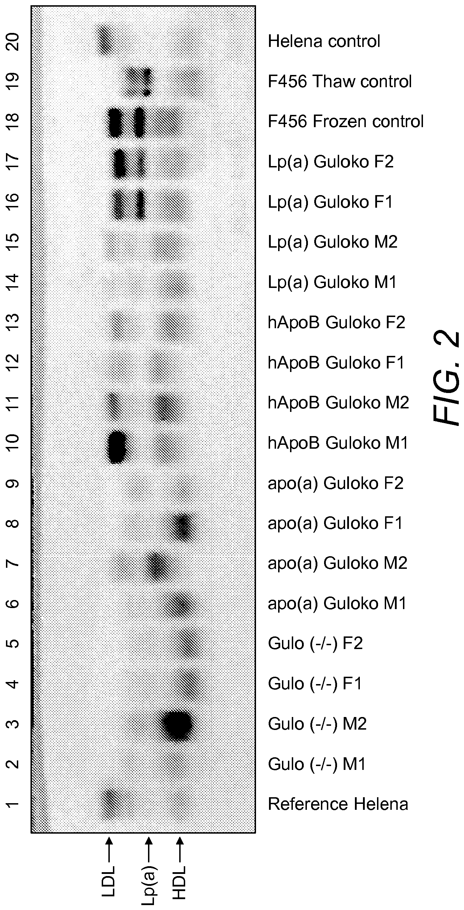

Tail clips were obtained from mouse under anesthesia and then shipped to Transnetyx where the following probe sets were designed and used for Real-Time PCR detection of genomic DNA presence or absence (primers used are shown below in the Tables 2, 3 and 4. Litters were genotyped via tail clip Taqman PCR at Transnetyx and those positive for genomic transgenes apo(a) and apoB-100, as well as homozygous knockout mutation for the L-gulonolactone-oxidase gene, GULO (-/-) , which indicates vitamin C synthesis defect, were selected and labeled as "Rath M Human Lipoprotein(a); Gulo (-/-)" founder mouse . In Fig.2 we show that the disulfide linked lipoprotein (a) is formed in the dual transgenic mouse and shown as Lp(a) GULOKO F1, F2, M1 and M2. Similarly, the lack of Lp(a) in apo(a)+ mouse without human apoB-100 expression and apo B-100 + mice without apo(a) expression is also shown.

**Table 2:GULO testing:**

| |
|---|
| **Gulo-1 KO Forward Primer:** CTAGTGTAGTCTAGGTGATAAGGATCAACT- Seq 1 |
| **Gulo-1 KO Reverse Primer:** CAGCTCAGAGAGAGAATGAATCACA- Seq 2 |
| **Reporter 1:** CTGACATCCCTTAGGAGTTC - Seq 3 |
| **Gulo-1 WT Forward Primer:** AGATGTGTTCCAGGCTGCAA- Seq 4 |
| **Gulo-1 WT Reverse Primer:** CACACACTGCAGGGTGACA - Seq 5 |
| **Reporter1:** CTGCCTGGGTGTTATC - Seq 6 |

Genotype Results Interpretation: Gulo-1 KO+, Gulo-1 WT+ = Hemizygous Vitamin C generating mouse. Gulo-1 KO-, Gulo-1 WT+ = Homozygous wild type Vitamin C generating mouse. Gulo-1 KO+, Gulo-1 WT- = Homozygous Vitamin C defective mouse.

Mouse homozygous for the knockout, GULO(-/-) mouse was selected for cross-breeding.

**Table 3 : B: Human apo(a) testing**

| |
|---|
| HuLPA-1 Tg (Human apo(a) transgene) Forward Primer: CACTACATTTTGTGCCAGAGATGGA - - Seq 7 |
| HuLPA-1 Tg Reverse Primer: CCCTGTCCTGAGGCTCCTTA - Seq 8 |
| Reporter 1: TCAGCAGCCCTCTTCC - Seq 9 |

Genotype Results Interpretation: + = Human apo(a) gene positive. - = Human apo(a) gene negative. Mouse positive for the transgene were selected for cross-breeding.

**Table 4: Human apo B-100 testing primers**

| |
|---|
| ApoB Tg (Human ApoB100 Transgene) Forward Primer: AGGTTTAACTCCTCCTACCTCCAA- - Seq 10 |
| ApoB Tg Reverse Primer: TGAGGGAGAGGGTTCCATCTT- - Seq 11 |
| Reporter 1: ACCAGATAACAGGAAGATATG - - Seq 12 |

Genotype Results Interpretation: + = Human apoB-100 gene positive. - = Human apoB-100 gene negative. Mouse positive for the transgene were selected for cross-breeding.

The genotype of the Lp(a); GULO (-/-) mouse is denoted as h apo(a)+; h apoB-100+; GULO(-/-). The mouse must continually be maintained on vitamin C supplementation as described above.

Confirming the transgene mouse generation at the level of protein: The presence of human apo(a) and human apo B-100 proteins in mouse sera was determined by ELISA in the serum drawn from the GULO (-/-) mouse, the apo(a)+ GULO(-/-) mouse, the apoB+ GULO (-/-) mouse, and the Lp(a)+ GULO (-/-) mouse.

**Table 5: Lp(a); GULOKO mice and hApoB; GULOKO mice express human ApoB via AssayPro ApoB ELISA**

| Sample name | ug/mL | ug/ml^{∗}20000 | mg/dL |
|---|---|---|---|
| gko m1 | 0 | | |
| gko m2 | 0.001153 | | |
| gko f1 | 0.000374 | | |
| gko f2 | -0.00036 | | |
| **Lp(a); gko m1** | 0.014717 | 294.3472224 | 29.4mg/dL |
| **Lp(a); gko m2** | 0.015867 | 317.346142 | 31.7mg/dL |
| **Lp(a); gko f1** | 0.041693 | 833.8570197 | 83.3mg/dL |
| **Lp(a); gko f2** | 0.041411 | 828.2183041 | 82.8mg/dL |
| apo(a); gko m1 | 0.000185 | | |
| apo(a); gko m1 | -0.00018 | | |
| apo(a); gko f1 | -0.00054 | | |
| apo(a); gko f2 | -0.00018 | | |
| **hApoB; gko m1** | 0.056842 | 1136.847637 | 114mg/dL |
| **hApoB; gko m2** | 0.020853 | 417.0655494 | 41.7mg/dL |
| **hApoB; gko f1** | 0.003275 | 65.5092541 | 6.6mg/dL |
| **hApoB; gko f2** | 0.017319 | 346.3860878 | 34.6mg/dL |
| Serum diluted 1:20,000 | | | |

The apo(a) protein was present in serum of both male and female mouse before puberty. Male mouse after puberty have significantly or completely repressed apo(a) protein expression due to elevated testosterone levels. Apo(a) expression in male mouse may be restored via castration, continuous infusion of growth hormone via osmotic pump, or by biochemical modulation by dietary, chemical, or biological inducers.

Human apo B-100 protein expression: The presence of human apoB-100 protein in serum was determined by ELISA in a serum drawn from the GULO (-/-) mouse, the apo(a); GULO(-/-) mouse, the apoB; GULO (-/-) mouse, and the Lp(a); GULO (-/-) mouse.

Presence of apoB-100 protein in mouse serum was determined by using Assaypro (St. Charles, MO) AssayMax Human Apolipoprotein enzyme immunoassay which is human apoB-100 specific and does not cross-react with mouse apoB-100, and/or with any other of the apolipoproteins (Apo AI, ApoC, ApoE).

Human apoB-100 was detected in the sera of hApoB-100; GULO (-/-) mouse, hApoB-100; apo(a); GULO(-/-) mouse, but not apo(a); GULO (-/-) mouse or GULO (-/-) mouse (Table 5).

Serum apo(a) protein was present in apo(a) gene containing GULO (-/-) mouse, apo(a) and human ApoB-100 gene containing GULO (-/-) mouse, but not GULO (-/-) mouse without the transgene nor in GULO (-/-) mouse with only human apo B-100 (Table 6)). These results confirm expression and translation of the human transgene apo(a) to serum protein apo(a).

**TABLE 6: Female and male apo(a) expression. Male mouse may have low expression because of high testosterone levels in blood.**

| Sample names | mg/dL apo(a) | | Comments |
|---|---|---|---|
| GKO m1 | 0.66168 | (below detection limit) | This test does not cross react with plasminogen or LDL. |
| GKO m2 | 0 | (below detection limit) | |
| GKO f1 | -0.33084 | (below detection limit) | No apo(a) detected in background GULOKO mice. |
| GKO f2 | -0.33084 | (below detection limit) | No apo(a) detected in hApoB; GULOKO mice. |
| Lp(a); gko m1 | 0.16542 | (below detection limit) | Extremely high apo(a) detected in apo(a); GULOKO female mice. |
| Lp(a); gko m2 | 0.16542 | (below detection limit) | No apo(a) detected in apo(a); GULOKO male mice. |
| Lp(a); gko f1 | 86.51466 | (#464) | Extremely high apo(a) detected in Lp(a); GULOKO female mice, either generation F1 or F2. |
| Lp(a); gko f2 | 79.4016 | (#110) | No apo(a) detected in Lp(a); GULOKO male mice, either F1 or F2. |
| apo(a); gko m1 | 0.33084 | (below detection limit) | |
| apo(a); gko m2 | -0.33084 | (below detection limit) | Sex steroid testosterone suppresses apo(a) production in these mice. |
| apo(a); gko f1 | 133.8248 | | Orchidectomy may have to be performed in order to express Lp(a). |
| apo(a); gko f2 | 156.1565 | | |
| hApoB; gko m1 | 0.16542 | (below detection limit) | (#110 - ApoB gene signal = 13.5, apo(a) gene signal = 3.7) |
| hApoB; gko m2 | 0 | (below detection limit) | (#464 - ApoB gene signal = 20.4, apo(a) gene signal = 8.5) |
| hApoB; gko f1 | -0.16542 | (below detection limit) | (#456 - ApoB gene signal = 18.8, apo(a) gene signal = 6.4) |
| hApob; gko f2 | 0.66168 | (below detection limit) | |

Human apo (a) protein expression: Presence of apo (a) protein in serum was determined by using the IBL International GmbH Lp(a) Enzyme immunoassay which is human apo (a) specific and does not cross-react with plasminogen or LDL. All known isoforms of apo(a) can be detected.

Human Lp(a) protein expression: The Lp(a) particles are composed of human apo(a) protein linked to human apo B-100 (the main protein of the LDL particle) by a disulfide bond.

SPIFE Cholesterol Profiling (FIG. 2): The presence of complete Lp(a) lipoprotein particles in the Lp(a)+ GULO(-/-) transgenic mouse serum was confirmed using the electrophoresis method with Helena (Beaumont, TX) SPIFE Cholesterol Profiling and Immunofixation electrophoresis (IFE).

The Lp(a)-cholesterol band runs at a specific migration distance in respect to LDL-cholesterol, and HDL-cholesterol, and it is found in human Lp(a)+; GULO(-/-) mouse sera, but not in the sera of GULO (-/-), human apo(a); GULO(-/-), or human apoB; GULO (-/-) mouse confirming that the presence of both human apoB-100 and human apo(a) are necessary to form complete Lp(a) particles in serum and that human apo(a) alone is insufficient to produce Lp(a) and it does not link to mouse LDL via disulfide bonds. In Fig. 2 the band closest to the top of the gel corresponds to LDL-cholesterol, and the band furthest from the top to HDL-cholesterol. The tight, middle bands located between the LDL and HDL bands which are present in lanes 16-18 represent the Lp(a)-cholesterol from three different female Lp(a); GULO(-/-) mice . These bands are missing from those GULO(-/-) mice not simultaneously expressing both human apo(a) and human apoB-100 transgenes. Lane 19 represents charge-mass shifts resulting from a 24 hour room temperature incubation of serum specimen #18, which indicates that a small shift in the particle migration may relate to lipoprotein oxidation.

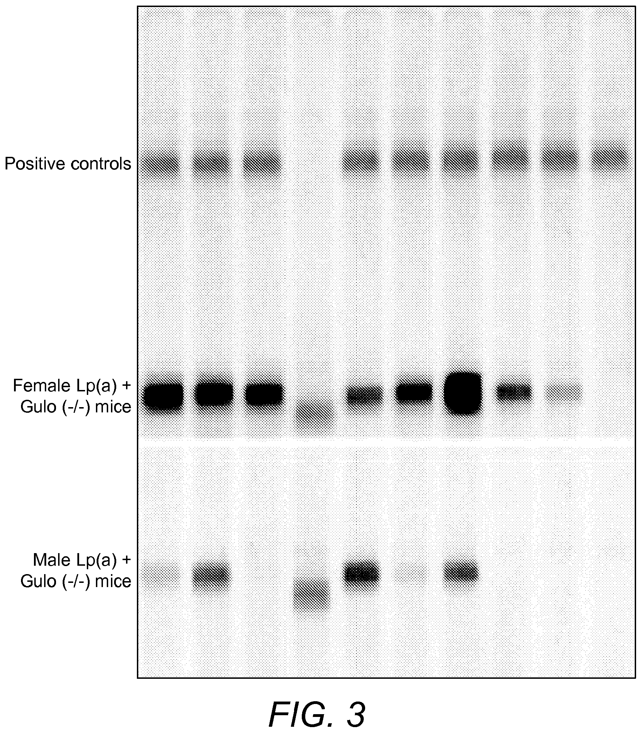

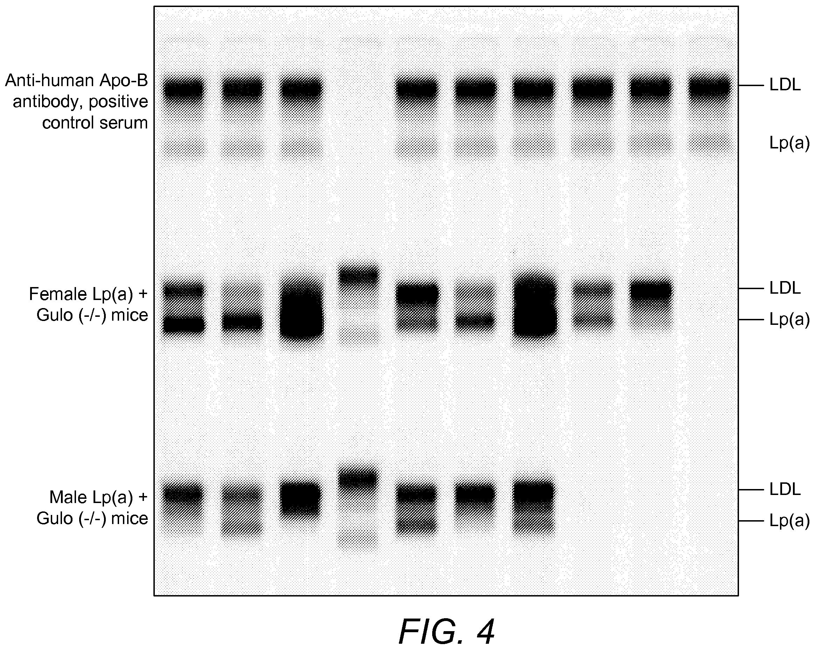

Immunofixation Electrophoresis (IFE) using the mouse sera was conducted on individual apo(a) (FIG. 3) containing particles and human apoB-100 containing particles (Fig.4) as well, using human specific apo(a) and apoB-100 antibodies at Health Diagnostic Laboratory, Inc. (Richmond, VA). The bands represent apo(a) and apoB-100 protein respectively in transgenic mouse and visualization of the same in serum derived from female and male mouse.

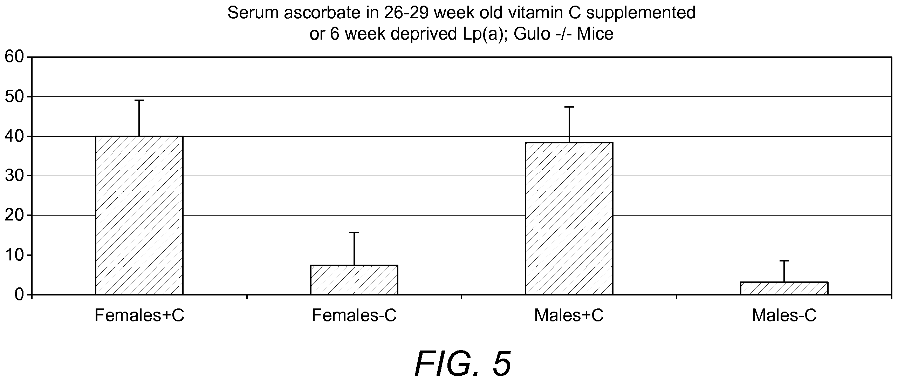

Confirming a lack of vitamin C production in the transgenic mouse strains: Serum level of ascorbate (vitamin C) in both GULO(-/-) mouse and a newly generated strain of Lp(a)+ GULO (-/-) depends on its dietary supplementation. Mouse kept on vitamin C deficient diet has a gradually diminishing serum concentration of vitamin C until it reaches zero or the mouse dies. Serum levels of vitamin C were obtained using the Biovision (Mountain View, CA) Ferric Reducing Ascorbate Assay (FRASC) Kit (Fig. 5).

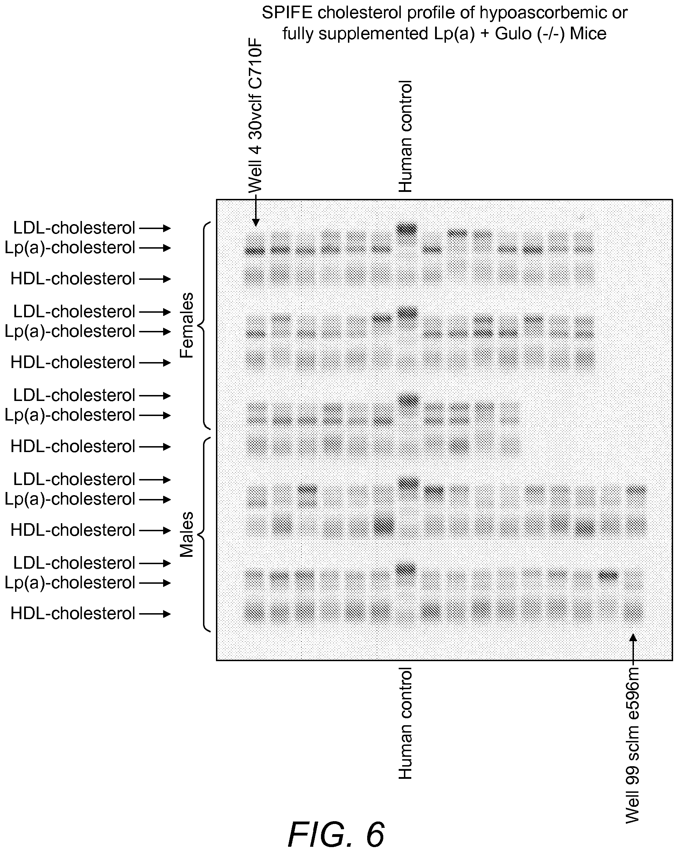

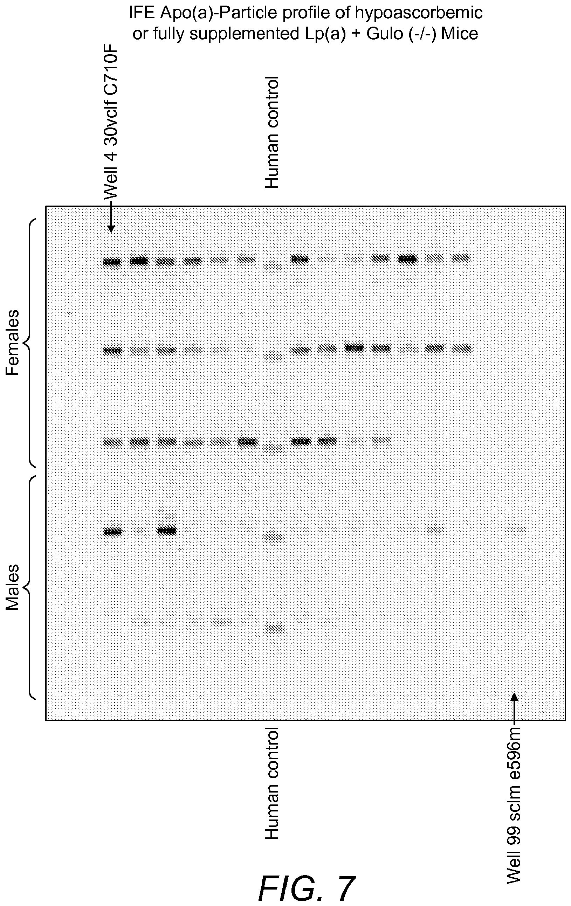

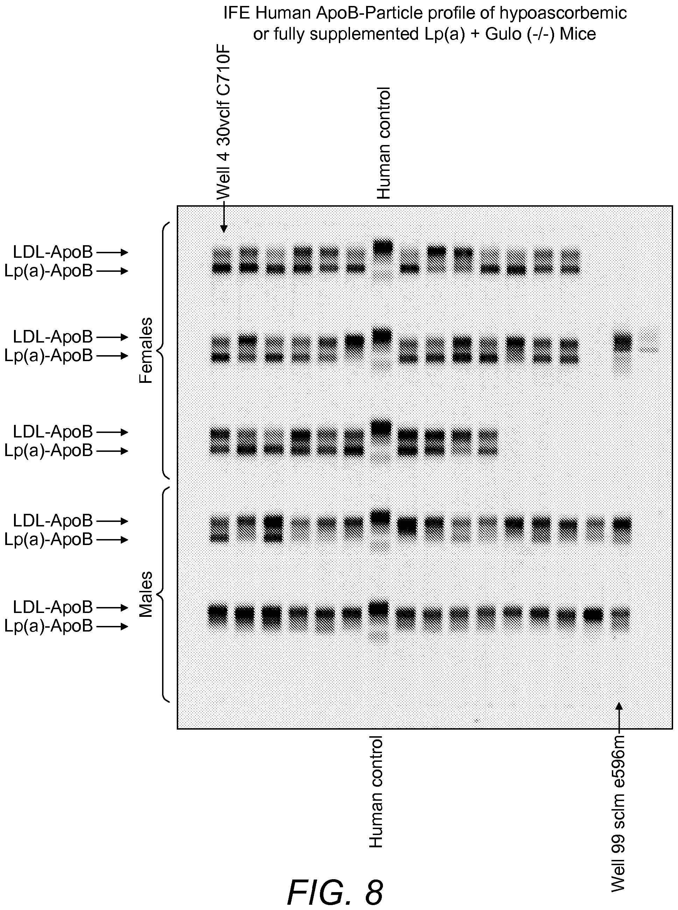

Lipoprotein-cholesterol, Apo(a) particle, and ApoB-100 particle Modulation (Fig. 6, Fig. 7, and Fig. 8): The analysis was conducted on mouse sera from the Lpa+ Gulo (-/-)mouse supplemented with either 30mg/L , 60mg/L or 150mg/L of ascorbic acid provided in drinking water in addition to 500ppm vitamin C provided in food (full supplementation). It was observed that the whole spectrum of lipoprotein cholesterols and/or lipoprotein particles could be modulated by dietary ascorbate alone. These data give additional particle number data and in conjunction with the lipoprotein cholesterol load data provided comprehensive confirmation of the presence of apo(a) protein, human apoB-100 protein, and disulfide linked Lp(a) in these mouse sera.

Sample order in Figures 6-8 correspond to the following key, with 30vc referring to 30mg/L Vitamin C group, 60vc referring to 60mg/L Vitamin C group, and sc referring to fully supplemented (150mg/L Vitamin C + 500ppm Vitamin C in food) control group. The first three wells of each row were not used.

**Table 7: Sample list for the wells in Fig. 6, 7 and 8.**

| **Well#** | **Group** | **Sample ID** | **Well#** | **Group** | **Sample ID** |
|---|---|---|---|---|---|
| 4 | 30vclf | e710f | 51 | sc1f | e731f |
| 5 | 30vc2f | e689f | 52 | sc1f | e812f |
| 6 | 30vc2f | e598f | 53 | sc1f | e832f |
| 7 | 30vc2f | e704f | 54 | sc2f | e778f |
| 8 | 30vc2f | e723f | 64 | 30vc2m | e697m |
| 9 | 30vc2f | e717f | 65 | 30vc2m | e698m |
| 10 | human control | | 66 | 30vc2m | e770m |
| 11 | 30vc1f | fell off 1 | 67 | 30vc2m | fell off |
| 12 | 30vc1f | e762f | 68 | 30vc2m | e695m |
| 13 | 30vc1f | e759f | 69 | 30vc1m | e683m |
| 14 | 30vc1f | e699f | 70 | human control | |
| 15 | 30vc1f | e706f | 71 | 30vc1m | e684m |
| 16 | 30vc1f | fell off 2 | 72 | 30vc1m | fell off |
| 17 | 60vc2f | e773f | 73 | 30vc1m | e693m |
| 24 | 60vc2f | ea315f | 74 | 30vc1m | e727m |
| 25 | 60vc2f | e771f | 75 | 60vc2m | e782m |
| 26 | 60vc2f | e694f | 76 | 60vc2m | fell off 2 |
| 27 | 60vc2f | e761f | 77 | 60vc2m | fell off 1 |
| 28 | 60vc2f | e768f | 78 | 60vc2m | e809m |
| 29 | 60vclf | e776f | 79 | 60vc2m | e738m |
| 30 | human control | | 84 | 60vc1m | e811m |
| 31 | 60vc1f | e777f | 85 | 60vc1m | e736m |
| 32 | 60vc1f | e780f | 86 | 60vc1m | fell off |
| 33 | 60vc1f | fell off | 87 | 60vc1m | e734m |
| 34 | 60vc1f | e733f | 88 | 60vc1m | e740m |
| 35 | 60vc1f | e784f | 89 | sc2m | fell off 2 |
| 36 | sc2f | e741f | 90 | human control | |
| 37 | sc2f | e814f | 91 | sc2m | e724m |
| 44 | sc2f | fell off 2 | 92 | sc2m | e605m |
| 45 | sc2f | fell off 1 | 93 | sc2m | e702m |
| 46 | sc2f | e781f | 94 | sc2m | fell off 1 |
| 47 | sc1f | e732f | 95 | sc1m | e705m |
| 48 | sc1f | e730f | 96 | sc1m | e718m |
| 49 | sc1f | e739f | 97 | sc1m | e701m |
| 50 | human control | | 98 | sc1m | e703m |
| | | | 99 | sc1m | e596m |

### INDUSTRIAL APPLICATION:

Crossbreeding a dual transgenic mouse to produce a human Lp(a) and not produce vitamin C due to lack of GULO (GULO-/-) gene using transgenic mouse having a first knockout strain, a second strain to make a third strain and using the first knockout strain and fourth strain to make a fifth strain, using the third strain and the fifth strain to make a dual transgenic mouse. Treating the dual transgenic mouse with a Lp(a)-modulating compounds in order to identify preventive and/or therapeutic approaches for a human Lp(a)-related diseases. The human Lp(a)-related disease is cardiovascular, inflammatory, infectious or degenerative in nature.

## Claims

1. A dual transgenic mouse having a gulonolactone oxidase gene knockout (GULO-/-), a functional human apolipoprotein B-100 gene (apo( B-100) +) and a functional human apolipoprotein (a) gene (apo (a) +) that produces human lipoprotein (a) and produces no Vitamin C, wherein said transgenic mouse is obtained by:
crossbreeding a first knockout strain mouse-and a second strain of transgenic mouse expressing a set of genes that are human in nature, wherein the first knockout strain mouse comprises a non-functional gulonolactone oxidase gene, GULO gene (GULO-/-), and produces no Vitamin C, wherein the second strain of mouse possesses a human apolipoprotein (a) gene (apo (a) +), to obtain a third strain of transgenic mouse, wherein the third strain of transgenic mouse expresses the human apo (a) gene (apo (a) +) and comprises a non-functional GULO gene (GULO-/-);
and crossbreeding the first knockout strain mouse comprising a non-functional GULO gene and a fourth strain of mouse possessing a human apolipoprotein B-100 gene (apo B-100 +), to obtain a fifth strain of mouse, wherein the fifth strain of mouse comprises a non-functional GULO gene (GULO-/-) and expresses the human apo B-100 gene (apo B-100 +); and
crossbreeding the third strain of mouse comprising a non-functional GULO gene (GULO-/-) and expressing the human apo (a) gene (apo (a) +), and the fifth strain of transgenic mouse comprising a non-functional GULO gene (GULO-/-) and possessing the human apo B-100 gene (apo B-100 +) to obtain the dual transgenic mouse comprising a non-functional GULO gene (GULO-/-) and which, simultaneously, produces human lipoprotein (a) (Lp(a)+) and produces no Vitamin C.

2. The dual transgenic mouse of claim 1, wherein said dual transgenic mouse expressing apolipoprotein (a) and apolipoprotein B-100, wherein said dual transgenic mouse produces human lipoprotein (a) particles and is unable for endogenous ascorbate synthesis.

## Patentansprüche

1. Dual-transgene Maus mit einem Gulonolactonoxidase-Gen-Knockout (GULO-/-), einem funktionellen menschlichen Apolipoprotein-B-100-Gen (apo( B-100) +) und einem funktionellen menschlichen Apolipoprotein-(a)-Gen (apo (a) +), das menschliches Lipoprotein (a) produziert und kein Vitamin C produziert, wobei die transgene Maus erhalten wird durch:
Kreuzen einer Maus eines ersten Knockout-Stamms und eines zweiten Stammes transgener Mäuse, der einen Satz von Genen exprimiert, die menschlicher Natur sind, wobei die Maus des ersten Knockout-Stamms ein nicht-funktionales Gulonolactonoxidase-Gen, GULO-Gen (GULO-/-), umfasst und kein Vitamin C produziert, wobei der zweite Mausstamm ein menschliches Apolipoprotein (a)-Gen (apo (a) +) besitzt, um einen dritten Stamm transgener Mäuse zu erhalten, wobei der dritte Stamm transgener Mäuse das menschliche apo (a)-Gen (apo (a) +) exprimiert und ein nicht-funktionales GULO-Gen (GULO-/-) umfasst;
und Kreuzen der Maus des ersten Knockout-Stamms, die ein nicht-funktionales GULO-Gen umfasst, und eines vierten Mausstamms, der ein menschliches Apolipoprotein B-100-Gen (apo B-100 +) besitzt, um einen fünften Mausstamm zu erhalten, wobei der fünfte Mausstamm ein nicht-funktionales GULO-Gen (GULO-/-) umfasst und das menschliche apo B-100-Gen (apo B-100 +) exprimiert; und
Kreuzen des dritten Mausstamms, der ein nicht-funktionales GULO-Gen (GULO-/-) und das menschliche apo (a)-Gen (apo(a)+) exprimiert, und des fünften Stammes der transgenen Maus, der ein nicht-funktionelles GULO-Gen (GULO-/-) umfasst und das menschliche apo B--100-Gen (apo B-100 +) besitzt, um die dual-transgene Maus zu erhalten, die ein nicht-funktionelles GULO-Gen (GULO-/-) umfasst und die gleichzeitig menschliches Lipoprotein (a) (Lp(a)+) produziert und kein Vitamin C produziert.

2. Dual-transgene Maus nach Anspruch 1, wobei die dual-transgene Maus Apolipoprotein (a) und Apolipoprotein B-100 exprimiert, wobei die dual-transgene Maus menschliche Lipoprotein (a)-Partikel produziert und zur endogenen Ascorbatsynthese unbefähigt ist.

## Revendications

1. Souris double transgénique possédant une inactivation de gène de gulonolactone oxydase (GULO-/-), un gène d'apolipoprotéine B-100 humain fonctionnel (apo(B-100) +) et un gène d'apolipoprotéine (a) humain fonctionnel (apo(a) +) qui produit une lipoprotéine (a) humaine et ne produit pas de vitamine C, ladite souris transgénique être obtenue par :
croisement d'une première souris à souche knock-out et d'une deuxième souche de souris transgénique exprimant un ensemble de gènes qui sont de nature humaine, la première souris à souche knock-out comprenant un gène de gulonolactone oxydase non fonctionnel, le gène GULO (GULO-/-), et ne produisant pas de vitamine C, la deuxième souche de souris possédant un gène d'apolipoprotéine (a) humain (apo (a)+), pour obtenir une troisième souche de souris transgénique, la troisième souche de souris transgénique exprimant le gène d'apo (a) humain (apo (a)+) et comprenant un gène GULO non fonctionnel (GULO-/-) ;
et croisement de la première souris à souche knockout comprenant un gène GULO non fonctionnel et une quatrième souche de souris possédant un gène d'apolipoprotéine B-100 humain (apo B-100 +), pour obtenir une cinquième souche de souris, la cinquième souche de souris comprenant un gène GULO non fonctionnel (GULO-/-) et exprimant le gène d'apo B-100 humain (apo B-100 +) ; et
croisement de la troisième souche de souris comprenant un gène GULO non fonctionnel (GULO-/-) et exprimant le gène de l'apo (a) humain (apo (a)+), et la cinquième souche de souris transgénique comprenant un gène GULO non fonctionnel (GULO-/-) et possédant le gène de l'apo B-100 humain (apo B-100 +) pour obtenir la souris double transgénique comprenant un gène GULO non fonctionnel (GULO-/-) et qui, simultanément, produit une lipoprotéine (a) humaine (Lp(a)+) et ne produit pas de vitamine C.

2. Souris double transgénique selon la revendication 1, ladite souris double transgénique exprimant l'apolipoprotéine (a) et 1'apolipoprotéine B-100, ladite souris double transgénique produisant des particules de lipoprotéine (a) humaine étant incapable pour la synthèse d'ascorbate endogène.
